# EUROPÄISCHE PATENTSCHRIFT

(11) **EP 2 313 134 B1**
(45) Veröffentlichungstag und Bekanntmachung des Hinweises auf die Patenterteilung: **19.02.2014**
(21) Anmeldenummer: 09777927.6
(22) Anmeldetag: 17.08.2009
(51) Int. Cl.: A61M 5/44, A61M 1/28

(54) **Dialysegerät enthaltend eine Kassette zur Förderung von Flüssigkeiten, insbesondere Dialyseflüssigkeiten**
Dialysis device with a cartridge for conveying fluids, particularly dialysis fluids
Dispositif de dialyse comprentant une cassette pour l'acheminement de liquides, en particulier, de liquides de dialyse

(30) Priorität: 18.08.2008 DE 102008038097
(43) Veröffentlichungstag der Anmeldung: 27.04.2011
(73) Patentinhaber: Fresenius Medical Care Deutschland GmbH, 61352 Bad Homburg (DE)
(72) Erfinder: HEDMANN, Frank, 97332 Volkach (DE); KLATTE, Stephan, 31582 Nienburg (Weser) (DE)
(74) Vertreter: Laufhütte, Dieter
(86) Internationale Anmeldenummer: PCT/EP2009/005956
(87) Internationale Veröffentlichungsnummer: WO 2010/020390

(56) Entgegenhaltungen:
- EP-A- 0 956 876
- EP-A- 1 195 171
- WO-A-00/16722
- US-A1- 2008 021 377
- US-A1- 2009 114 582
- US-A1- 2009 124 963

## Beschreibung

Die Erfindung betrifft ein Dialysegerät enthaltend eine Kassette zur Förderung von Flüssigkeiten, insbesondere Dialyseflüssigkeiten nach dem Oberbegriff des Anspruchs 1.

Bereits aus der EP 0 956 876 sind entsprechende Kassetten zur Förderung von Dialyseflüssigkeit bekannt, die Anschlußelemente zur Konnektion von Lösungsbeuteln und zum Patienten und dem Dialysegerät führenden Leitungen mit wenigstens einer Pumpkammer mit Zu- und Ablauf sowie mit Leitungen zur Führung der zugeführten und geförderten Flüssigkeit aufweisen, wobei die Wandungen der Leitungen wenigstens abschnittsweise derart ausgeführt sind, dass die Leitungen durch das Aufbringen einer auf die Wandungen wirkenden Druckkraft verschließbar sind. Diese vorbekannte Kassette enthält eine Heizvorrichtung zum Aufheizen der Dialyseflüssigkeit auf eine gewünschte Soll-Temperatur enthalten.

Derartige Kassetten werden beispielsweise im Bereich der Peritonealdialyse und hier insbesondere in der Peritonealdialyse, bei der das Peritoneum mit Hilfe einer als "Cycler" bezeichneten Maschine befüllt und entleert wird und bei der die Flußsteuerung über ein Kassettensystem erfolgt, angewandt.

In der Kassette wird die Dialysierflüssigkeit auf die gewünschte Soll-Temperatur erwärmt und anschließend in das Peritoneum infundiert.

Kommt es nun zu einem unerwarteten Anhalten des Cyclers, bei dem die Flüssigkeitszufuhr zum Patienten unterbrochen werden muß, wird das nunmehr nicht mehr in der Kassette umgewälzte Dialysat aufgrund der Trägheit der Heizvorrichtung über die Soll-Temperatur hinaus erhitzt. Aufgrund der Überschreitung der Soll-Temperatur kann nach erneutem Start des Cyclers das überhitzte Dialysat nicht mehr zur Infusion in den Patienten verwendet werden. Bei bekannten Systemen wird das Dialysat daher vollständig verworfen. Dies führt allerdings zu einem unerwünschten und letztendlich kostenintensiven Mehrverbrauch.

Es ist daher die Aufgabe der vorliegenden Erfindung, ein Dialysegerät, enthaltend eine Kassette zur Förderung von Flüssigkeit nach dem Oberbegriff des Anspruchs 1 derart fortzubilden, dass eine gegebenenfalls überhitzte Flüssigkeit auf eine gewünschte Soll-Temperatur zurückführbar ist, ohne diese verwerfen zu müssen.

Erfindungsgemäß wird diese Aufgabe durch die Kombination der Merkmale des Anspruchs 1 gelöst. Demnach wird ein Dialysegerät enthaltend eine Kassette zur Förderung von Flüssigkeiten, insbesondere Dialyseflüssigkeiten, geschaffen, die Anschlußelemente zur Konnektion von Lösungsbeuteln und zum Patienten oder Dialysegerät führende Leitungen aufweist, wobei sie wenigstens eine Förderkammer mit einem Zu- und Ablauf aufweist, weiter Leitungen zur Führung der zugeführten und geförderten Flüssigkeiten, weiterhin Ventile zum abschnittsweisen Verschließen der Leitungen und eine Heizvorrichtung zum Aufheizen der in den Leitungen befindlichen Flüssigkeit auf eine vorgebbare Soll-Temperatur. Erfindungsgemäß ist in einem derartigen Dialysegerät eine Steuerung vorgesehen, über die die über die Soll-Temperatur aufgeheizte Flüssigkeit so lange in der Kassette geführt wird, bis die Soll-Temperatur wieder erreicht ist.

Hierdurch kann die Flüssigkeit wieder verwendet werden. Die überschüssige Wärme kann durch die entsprechende Umwälzung innerhalb der Kassette an die sie umgebende Kassette übertragen werden.

Weiterhin ist mindestens ein Temperatursensor zur Messung der Temperatur der Flüssigkeit in der Kassette vorgesehen.

Erfindungsgemäß sind zwei Pumpkammern vorgesehen, zwischen denen das Medium bis zum Erreichen der Soll-Temperatur veschiebbar ist.

Vorteilhaft ist die Heizvorrichtung als Durchlauferhitzer ausgestaltet.

Die Erfindung betrifft weiterhin ein Verfahren gemäß Anspruch 3 zur Temperierung einer Flüssigkeit in einer Kassette, wie sie zuvor beschrieben wurde. Erfindungsgemäß wird hier die Flüssigkeit zwischen den beiden Pumpenkammern verschoben, bis die Soll-Temperatur erreicht ist.

Bevorzugte Ausgestaltungen des erfindungsgemäßen Verfahrens ergeben sich aus den sich an den Anspruch 3 anschließenden Unteransprüchen.

Demnach wird bei Überschreiten der Soll-Temperatur der Flüssigkeit die Leitung, an die die Flüssigkeit abführende Leitung angeschlossen ist, geschlossen. Gleichzeitig wird die Heizleistung der Heizvorrichtung reduziert. Die Flüssigkeit wird durch eine der Pumpkammern aus der Heizvorrichtung in die jeweils andere Pumpkammer gepumpt.

Für den Fall, dass sich in der Pumpkammer, in die die überhitzte Flüssigkeit geführt wird, ein hoher Anteil kalter Flüssigkeit bereits befindet, wird die gemischte Flüssigkeit, also die Flüssigkeit mit der sich ergebenen Mischtemperatur, im Rückwärtsbetrieb durch die Heizvorrichtung in die jeweils andere Pumpkammer gefördert, um dabei gleichzeitig die Heizvorrichtung abzukühlen.

Sollte dagegen aufgrund der Zumischung der überhitzten Flüssigkeit in die Pumpkammer eine in ihrer Mischtemperatur überhitze Flüssigkeit resultieren, wird die gemischte Flüssigkeit im Vorwärtsbetrieb durch die Heizvorrichtung in die jeweils andere Pumpkammer gefördert, um die überschüssige Wärme in der Kassette zu verteilen.

Weitere Merkmale, Einzelheiten und Vorteile der Erfindung ergeben sich aus der anschließenden Beschreibung eines bevorzugten Ausführungsbeispiels der Erfindung.

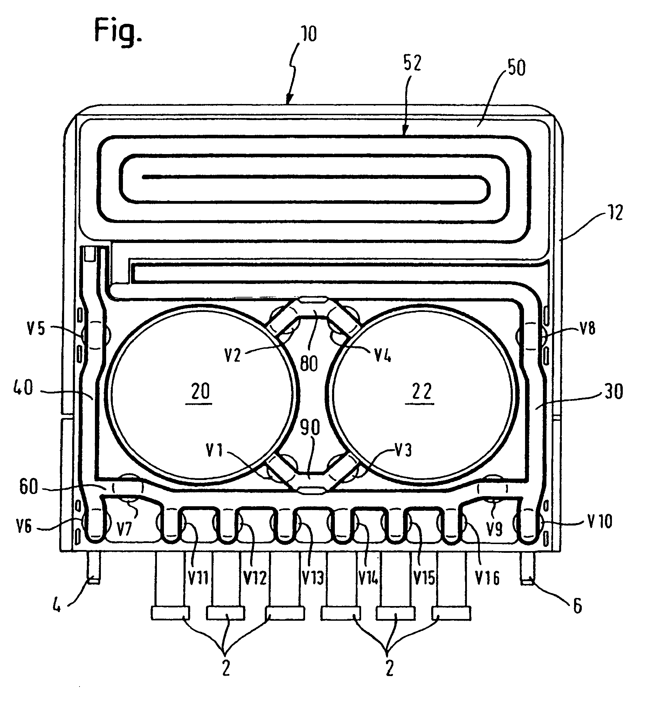

Die einzige Figur zeigt eine Draufsicht auf eine Ausführungsform der erfindungsgemäßen Kassette.

Die Figur zeigt eine Kassette 10, die als Disposable ausgeführt ist und die in eine entsprechend gestaltete Aussparung oder Aufnahme eines Dialysegerätes, vorteilhaft eines Cyclers einer Peritonealdialysevorrichtung, eingesteckt werden kann. Die Bezugszeichen 2, 4, 6 kennzeichnen Anschlußelemente zur Konnektion von Lösungsbeuteln, von zum Patienten oder zum Dialysegerät führenden Leitungen oder auch Drainageleitungen. An den Anschlüssen 4 und 6 sind zwei (nicht dargestellte) fest angebrachte Schläuche angeordnet, von denen einer den Patientenschlauch und der andere den Drainageschlauch darstellt. Die Anschlußelemente 2 dienen der durch den Bediener durchzuführenden Konnektion von beispielsweise Lösungsbeuteln oder anderen Medikamentenbehältern.

Die Kassette 10 umfaßt einen Grundkörper 12, der aus Kunststoff besteht und in Spritzgußtechnik oder auch Tiefziehtechnik herstellbar ist. In dem Grundkörper 12 erstrecken sich Ausnehmungen sowie Kanäle, die zum Teil die Wandungen von zwei nebeneinander angeordneten Förderkammern 20, 22 sowie der der Kassette angeordneten Leitungen (z. B. 30, 40, 50, 60) bilden. Zwischen den Pumpkammern 20 und 22 sind ebenfalls Leitungen 80 bzw. 90 ausgebildet.

Im Bereich der Leitungen 50 ist ein Durchlauferhitzer 52 als Heizvorrichtung verwirklicht.

Ansonsten entsprechen der Aufbau der Kassette und die Funktionsweise im wesentlichen derjenigen gemäß der EP 0 956 876 B1.

Zur Steuerung der zugeführten bzw. geförderten Flüssigkeit in den Leitungen 30, 40, 50, 60 dienen Ventile V1, V2, V3, V4, V5, V6, V7, V8, V9, V10, V11, V12, V13, V14, V15 sowie V16. Diese sind ebenfalls, wie in der EP 0 956 876 B1 beschrieben, über pneumatisch, hydraulisch oder auch mechanisch angesteuerte Ventilstößel verschließbar. Bei üblicher Funktionsweise der in der Figur dargestellten Vorrichtung wird Flüssigkeit von der Pumpkammer 22 durch den Durchlauferhitzer 52 in Richtung zur die Flüssigkeit abfördernden Leitung, die am Anschluß 4 in nicht näher dargestellter Art und Weise anschließt, gefördert. Damit fließt die Flüssigkeit von der Pumpkammer 22 über die Leitung 80 nach Öffnung des Ventils V4 durch die Leitung 50 im Durchlauferhitzer 52 und über das geöffnete Ventil V5 sowie das geöffnete Ventil V6. Gleichzeitig wird die Pumpkammer 20 mit frischer Flüssigkeit, beispielsweise über die geöffneten Ventile V11 und V1 versorgt, während natürlich gleichzeitig die Ventile V7, V3 und V2 sowie V9 geschlossen sind.

Ergeben sich nun Probleme in der Leitung, beispielsweise ein Verschluß der am Anschluß 4 anschließenden Leitungen zum Patienten, kann die im Durchlauferhitzer 52 befindliche Flüssigkeit aufgrund eines Überschwingens der Aufheizregelung soweit überhitzt werden, dass die Flüssigkeit nicht mehr in das Peritoneum infundiert werden kann.

Bei dem vorgenannten Überhitzen der im Durchlauferhitzer 52 befindlichen Flüssigkeit wird über eine hier nicht näher dargestellte Steuerung, die außerhalb der Kassette, beispielsweise im Cycler, angeordnet ist, das Ventil V6 geschlossen und die Heizleistung im Durchlauferhitzer 52 reduziert. Gleichzeitig wird in der Pumpkammer 20 die Befüllung mit frischer Lösung eingestellt. Hierzu wird das Ventil V11 geschlossen. Dafür wird nun über die Pumpkammer 22 die zu warme Lösung durch den Durchlauferhitzer 52 in die Pumpkammer 20 gepumpt, wobei hier das Ventil V4, das Ventil V5, das Ventil V7 und das Ventil V1 geöffnet ist. Der Pumpenhub der Pumpkammer 22 wird beendet, wenn die Pumpkammer 20 gefüllt ist oder die Pumpkammer 22 entleert ist.

In Abhängigkeit von dem Verhältnis der in der Pumpkammer 20 enthaltenden Flüssigkeit zu der in die Pumpkammer 20 hineingeförderten warmen Flüssigkeit ergeben sich nun grundsätzlich zwei Möglichkeiten zur Abkühlung der Flüssigkeit.

Bei der ersten Alternative wird davon ausgegangen, dass ein hoher Anteil von kalter Flüssigkeit in der Pumpkammer 20 vorhanden ist. In diesem Fall wird die vergleichsweise durch die Mischung der Flüssigkeiten kühlere gemischte Flüssigkeit in die Pumpkammer 22 über den Durchlauferhitzer 52 im Rückwärtsbetrieb gefördert, in dem hier über das geöffnete Ventil V1, das geöffnete Ventil V7, das geöffnete Ventil V5 und das geöffnete Ventil V4 die Flüssigkeit in die Pumpkammer 22 zurückgepumpt wird. Durch die vergleichsweise kühlere Flüssigkeit wird der partiell überhitzte Durchlauferhitzer zusätzlich abgekühlt.

Befindet sich nun ein recht hoher Anteil von warmer Flüssigkeit in der Pumpkammer 20, wird diese Flüssigkeit mit recht hoher Mischtemperatur in die Pumpkammer 22 über den Durchlauferhitzer 52 im Vorwärtsbetrieb gefördert, indem hier von der Pumpkammer 1 aus die Flüssigkeit über das geöffnete Ventil V2, das geöffnete Ventil V5, das geöffnete Ventil V7 und das geöffnete Ventil V3 in die Pumpkammer 22 geleitet wird. Bei dieser Variante wird die überschüssige Wärme der überhitzten Flüssigkeit in der Kassette verteilt, so dass die überhitzte Flüssigkeit auf die Soll-Temperatur abgesenkt werden kann.

## Patentansprüche

1. Dialysegerät enthaltend eine Kassette (10) zur Förderung von Flüssigkeiten, insbesondere Dialyseflüssigkeiten, mit Anschlusselementen (2, 4, 6) zur Konnektion von Lösungsbeuteln und zum Patienten oder Dialysegerät führenden Leitungen, auf der wenigstens eine Förderkammer mit Zu- und Ablauf, Leitungen zur Führung der zugeführten und geförderten Flüssigkeiten, Ventile (V1-V16) zum abschnittsweisen Verschließen der Leitungen, einer Heizvorrichtung (52) zum Aufheizen der in den Leitungen befindlichen Flüssigkeit auf eine vorgebbare Solltemperatur angeordnet sind, **dadurch gekennzeichnet dass**, die
über die Solltemperatur aufgeheizte Flüssigkeit innerhalb der Kassette geführt wird, wobei mindestens ein Temperatursensor vorgesehen ist und zwei Pumpenkammern (20, 22), zwischen denen die Flüssigkeit mittels einer Steuerung bis zum Erreichen der Solltemperatur verschoben wird.

2. Dialysegerät nach Anspruch 1, **dadurch gekennzeichnet, dass** die Heizvorrichtung ein Durchlauferhitzer ist.

3. Verfahren zur Temperierung einer Flüssigkeit in einer nach einem der voran gehenden Ansprüche ausgestalteten Kassette, **dadurch gekennzeichnet, dass** die Flüssigkeit zwischen den beiden Pumpkammern verschoben wird, bis die Solltemperatur erreicht ist.

4. Verfahren nach Anspruch 3, **dadurch gekennzeichnet, dass** bei Überschreiten der Solltemperatur der Flüssigkeit die Leitung, an die die Flüssigkeit abführende Leitung angeschlossen ist, verschlossen wird, dass die Heizleistung der Heizvorrichtung reduziert wird und dass die Flüssigkeit durch eine der Pumpkammern aus der Heizvorrichtung in die jeweils andere Pumpkammer gepumpt wird.

5. Verfahren nach Anspruch 4, **dadurch gekennzeichnet, dass** für den Fall, dass sich in der Pumpkammer, in die die überhitzte Flüssigkeit eingeführt wird, ein hoher Anteil kalter Flüssigkeit befindet, die gemischte Flüssigkeit im Rückwärtsbetrieb durch die Heizvorrichtung in die jeweils andere Pumpkammer gefördert wird, um die Heizvorrichtung dadurch abzukühlen.

6. Verfahren nach Anspruch 4, **dadurch gekennzeichnet, dass** für den Fall, dass sich in der Pumpkammer, in die die überhitzte Flüssigkeit eingeführt wird, ein hoher Anteil von zu heißer Flüssigkeit befindet, die gemischte Flüssigkeit im Vorwärtsbetrieb durch die Heizvorrichtung in die jeweils andere Pumpkammer gefördert wird, um die überschüssige Wärme in der Kassette zu verteilen.

## Claims

1. A dialyzer containing a cassette (10) for the conveying of liquids, in particular of dialysis liquids, having connection elements (2, 4, 6) for the connection of solution bags and of lines leading to the patient or to the dialyzer, on which cassette at least one conveying chamber with an inflow and an outflow, lines for the conducting of the supplied and conveyed liquids, valves (V1-V16) for the section-wise closing of the lines, a heating apparatus (52) for the heating of the liquid present in the lines to a presettable desired temperature are arranged, **characterized in that**
the liquid heated above the desired temperature is conducted in the cassette, wherein at least one temperature sensor is provided and two pump chambers (20, 22) are provided between which the liquid is displaced by means of a control up to the reaching of the desired temperature.

2. A dialyzer in accordance with claim 1, **characterized in that** the heating device is a continuous-flow heater.

3. A method for the temperature setting of a liquid in a cassette designed in accordance with one of the preceding claims, **characterized in that** the liquid is displaced between the two pump chambers until the desired temperature has been reached.

4. A method in accordance with claim 3, **characterized in that**, when the desired temperature of the liquid is exceeded, the line to which the line draining the liquid is connected is closed; **in that** the heating power of the heating device is reduced; and **in that** the liquid is pumped by one of the pump chambers from the heating device into the respective other pump chamber.

5. A method in accordance with claim 4, **characterized in that** in the event a high proportion of cold liquid is present in the pump chamber into which the overheated liquid is introduced, the mixed liquid is conveyed through the heating device into the respective other pump chamber in reverse operation in order thereby to cool down the heating device.

6. A method in accordance with claim 4, **characterized in that** in the event a high proportion of liquid which is too hot is present in the pump chamber into which the overheated liquid is introduced, the mixed liquid is conveyed through the heating device into the respective other pump chamber in forward operation to distribute the excess heat in the cassette.

## Revendications

1. Dispositif de dialyse comprenant une cassette (10) pour l'acheminement de liquides, en particulier de liquides de dialyse, comportant des éléments de raccordement (2, 4, 6) pour le raccordement de poches de solution et de tuyaux conduisant au patient ou au dispositif de dialyse, sur laquelle sont agencés au moins une chambre d'acheminement avec entrée et sortie, des tuyaux pour conduire les liquides alimentés et acheminés, des valves (V1-V16) destinées à fermer les tuyaux par sections, un dispositif de chauffage (52) pour chauffer le liquide se trouvant dans les tuyaux à une température de consigne pouvant être prédéfinie, **caractérisé en ce que**
du liquide chauffé au-dessus de la température de consigne est conduit à l'intérieur de la cassette, au moins un capteur de température étant prévu ainsi que deux chambres de pompage (20, 22) entre lesquelles le liquide est déplacé au moyen d'une commande jusqu'à ce que la température de consigne soit atteinte.

2. Dispositif de dialyse selon la revendication 1, **caractérisé en ce que** le dispositif de chauffage est un réchauffeur en continu.

3. Procédé de régulation de température d'un liquide dans une cassette réalisée selon l'une des revendications précédentes, **caractérisé en ce que** le liquide est déplacé entre les deux chambres de pompage jusqu'à ce que la température de consigne soit atteinte.

4. Procédé selon la revendication 3, **caractérisé en ce qu'**en cas de dépassement de la température de consigne du liquide, le tuyau auquel le tuyau évacuant le liquide est raccordé, est fermé, **en ce que** la puissance de chauffage du dispositif de chauffage est réduite et **en ce que** le liquide est pompé hors du dispositif de chauffage à travers l'une des chambres de pompage dans l'autre chambre de pompage respective.

5. Procédé selon la revendication 4, **caractérisé en ce que** dans le cas où une part élevée de liquide froid se trouve dans la chambre de pompage dans laquelle le liquide surchauffé est introduit, le liquide mélangé est acheminé en marche arrière à travers le dispositif de chauffage dans l'autre chambre de pompage respective pour refroidir ainsi le dispositif de chauffage.

6. Procédé selon la revendication 4, **caractérisé en ce que** dans le cas où une part élevée de liquide trop chaud se trouve dans la chambre de pompage dans laquelle le liquide surchauffé est introduit, le liquide mélangé est acheminé en marche avant à travers le dispositif de chauffage dans l'autre chambre de pompage respective pour répartir la chaleur excessive dans la cassette.
